# EUROPEAN PATENT SPECIFICATION

(11) **EP 4 171 388 B1**
(45) Date of publication and mention of the grant of the patent: **11.09.2024**
(21) Application number: 21732312.0
(22) Date of filing: 15.06.2021
(51) Int. Cl.: A61B 10/00, B01L 3/00, B65D 51/28

(54) **BIOPSY CONTAINER ASSEMBLY**
BIOPSIEBEHÄLTERANORDNUNG
ENSEMBLE RÉCIPIENT DE BIOPSIE

(30) Priority: 24.06.2020 IT 202000015235
(43) Date of publication of application: 03.05.2023
(73) Proprietor: Santoni, Stefano, 60030 San Marcello (AN) (IT)
(72) Inventor: GUERCIO, Giorgio, 60025 LORETO (AN) (IT)
(74) Representative: Cutropia, Gianluigi
(86) International application number: PCT/EP2021/066145
(87) International publication number: WO 2021/259710

(56) References cited:
- WO-A1-2012/171529
- WO-A1-2016/079611
- WO-A1-2018/078516
- WO-A2-2011/139026
- DE-A1- 102010 029 259
- GB-A- 2 482 211
- KR-Y1- 200 490 214
- US-A1- 2017 129 666

## Description

The present patent application for industrial invention relates to a biopsy container assembly.

As it is known, in order to perform a biopsy, a histological or bioptic sample is taken from the patient and is brought to a testing center that will perform the biopsy or histopathological analysis.

For the transportation of such histological samples, containers that contain formaldehyde wherein the sample is immersed for preservation purposes are known. However, the exposure of the operator to the vapors exhaled from formaldehyde involves health risks. In fact, it has been proved that formaldehyde is carcinogenic and has several side effects.

Various types of container assemblies are known in order to protect the operator from the exposure to formaldehyde fumes.

WO2012171529 discloses a container assembly comprising a container suitable for containing a tissue sample and a top member wherein a preservation liquid is contained and sealed with a membrane. A puncturing member is mounted in the top member and can be actuated by the user for breaking the membrane when the top member is closed on the container. In view of the above, the liquid passes through the membrane and flows from the top member to the container in order to cover the sample. Such a type of device of the prior art is impaired by some drawbacks. In fact, it must be considered that the tissue sample is generally sticky and tends to adhere to the bottom of the container. Consequently, if the container is turned upside down, the liquid flows into the top member, and the sample is no longer immersed in the liquid, sticking to the bottom of the container. Therefore such containers must be transported in a perfectly upright position.

A second type of container assembly is known, which comprises a first container suitable for containing a tissue sample and a second container that contains a preservation liquid. The first and the second container have walls provided with two holes and are disposed in opposite position. The two containers can be moved relative to each other in order to go from a closed position, wherein the holes in the two walls are not in communication and the preservation liquid is confined in the second container, to an open position, wherein the holes in the two walls are in communication and the preservation liquid can flow from the second container to the first container in order to immerse the sample. Also such a type of device of the prior art is impaired by drawbacks. In fact, the liquid contained in the second container is not perfectly sealed when the container assembly is in the closed position. Moreover, the use of the two containers is not particularly easy for the operator because the containers need to be moved in such a way to center the holes of the walls in order to open the container assembly. Additionally, the operator must remember to close the container assembly to prevent the preservation liquid from re-entering the second container if the biopsy container assembly is turned upside down.

Biopsy container assemblies filled with formaldehyde and with an oily liquid that forms a surface barrier on the formaldehyde so as to inhibit exhalations are also known. Such a type of containers is not perfectly safe and reliable.

The container assemblies of the prior art have the following problems:
- they cannot be used for all types of biopsies because of the risk of dispersion of small biopsies in the empty spaces of the container and/or in the slots of the cap, resulting in the loss of the biopsy;
- formalin can fall from the cap into the container due to the provision of very narrow slots;
- the handling of the parts of the container assemblies is complicated.

In order to protect the operator from formalin fumes, the container assemblies available on the market have a complicated structure that makes the insertion and the extraction of the biopsy sample difficult, with the risk of losing the biopsy. Consequently, the most practical container assembly is the standard container assembly that exposes the operator to formalin exhalations.

The biopsy container assemblies of the prior art are designed to protect the user from the fumes emitted by formalin - a tissue fixative reagent classified by IARC as carcinogenic - after opening such a container assembly. In order to be effective, formalin must be "ready to use", that is to say must have the correct concentration in order not to alter the correct chemical balance of interaction with the tissue cells. Such container assemblies contain a quantity of formalin in a more or less concentrated form that is contained under the cap of the container and is sealed with a membrane, whereas a quantity of buffer solution, which is inert in terms of carcinogenicity, is inserted in the container.

By means of a mechanism that breaks the membrane when the cap is closed, formalin is released in concentrated form, falling by gravity into the buffer solution and being diluted at the correct concentration of ready-to-use formalin.

Various rupturing mechanisms of the membrane are known, which create different types of ruptures on the membrane. Such rupturing mechanisms can have larger or smaller holes for the passage of formalin. In all cases, formalin falls in the container by gravity. Obviously, the larger the holes, the easier the fall of the concentrated formalin will be.

The mixture of ready-to-use formalin and buffer solution must have a correct formalin concentration. It is therefore necessary that all the concentrated formalin falls in the container where the buffer solution is contained.

It must be considered that the membrane rupturing mechanism (puncturing) creates fragmented membrane residues of various shapes that can hold the concentrated formalin, preventing it from completely falling by gravity.

Moreover, if the holes in the membrane are very small, a meniscus effect of the liquid molecules on the holes is created, which prevents the natural passage of the liquid because the small holes create a barrier against the fall by gravity.

Considering that the tissue samples introduced in the container always have a small, if not a very small size, even at the level of cellular size, the tendency is to create small holes in the membrane in order to prevent said small tissue samples from passing, along with formalin, through the holes of the membrane, returning to the compartment of the cap, if the container is turned upside down. If the sample passes in the compartment of the cap and the container is put in vertical position again, the formalin will fall again, but there is no guarantee that the sample will fall because it may get stuck in the fragmented membrane residues generated by the rupturing mechanism. In such a circumstance, the sample would be lost and it would be impossible to perform a correct histological test.

Therefore, the provision of larger holes will facilitate the fall of the concentrated formalin, but in such a case there will be a greater possibility of reflux in the cap and a greater risk of non-return of the sample if the container is turned upside down.

On the contrary, the provision of smaller holes will prevent the passage of small samples, but in such a case the small holes would prevent the fall of the concentrated formalin from the cap into the container by gravity (due to the meniscus effect of the liquid molecules on the holes).

GB2482211 discloses a dispensing system for a container according to the preamble of claim 1.

US2017/129666 discloses a liquid dispensing cap for a fluid container including a closure to install the cap on a neck of a fluid container, a liquid storage chamber, a seal applied over an opening to the chamber, a sliding mechanism that moves the seal against a cutter installed in a fixed position adjacent the seal to thereby release a liquid stored in the liquid storage chamber from the cap through the closure and into the fluid container.

DE102010029259 discloses a closure device for dispensing a substance into a container having an attachment part connectable with a container opening and a lid part connectable with the attachment part. A safety device prevents the two parts, which together define a substance chamber, from being removed from each other. A bottom of the substance chamber forms a component of the attachment part. The lid part has a perforation component for opening the bottom. Displacement of said component is performed by rotating the attachment part relative to the lid part along a thread, which has an attachment part thread component and a lid part thread component.

The purpose of the present invention is to eliminate the drawbacks of the prior art by disclosing a biopsy container assembly that is capable of preserving a bioptic sample effectively and safely for the user's health.

Another purpose of the present invention is to disclose such a biopsy container assembly that is practical, reliable, and easy to make and use.

These purposes are achieved according to the invention with the characteristics of the independent claim 1.

Advantageous embodiments of the invention appear from the dependent claims.

Additional features of the invention will be clearer from the following detailed description, which refers to a merely illustrative, not limiting embodiment, shown in the appended figures, wherein:
Fig. 1 is an exploded perspective view of the biopsy container assembly according to the invention;
Fig. 2 is an axial view of the biopsy container assembly of Fig. 1;
Figs. 3 and 4 are two perspective views of a crown of the biopsy container assembly of Fig. 1, taken from a different angle;
Fig. 5 is a perspective view of a cap of the biopsy container assembly of Fig. 1;
Fig. 6 is an axial view of the biopsy container assembly in closed condition before use;
Fig. 7 is an axial view of the biopsy container assembly during a closing of the cap, after introducing a bioptic sample and after turning the crown upside down;
Fig. 8 is a view of the biopsy container assembly after the closing of the cap, which has determined a rupture of a sealing film;
Fig. 9 is an exploded perspective view of a second embodiment of a biopsy container assembly;
Fig. 10 is an axial view of the biopsy container assembly of Fig. 2;
Fig. 11 is an axial view of the biopsy container assembly of Fig. 10 in assembled condition.

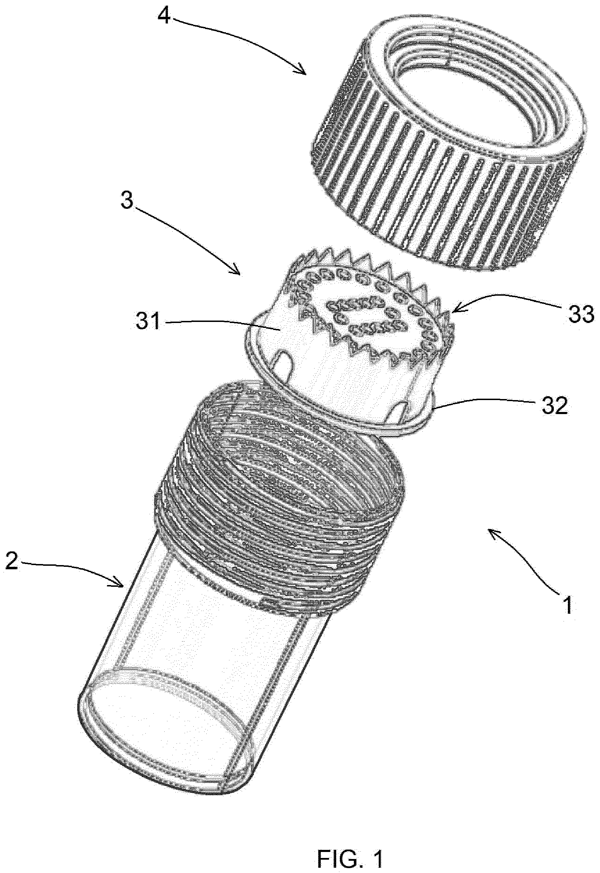

With reference to Figs. 1 to 8, the biopsy container assembly of the invention is described, which is generally indicated with reference numeral 1.

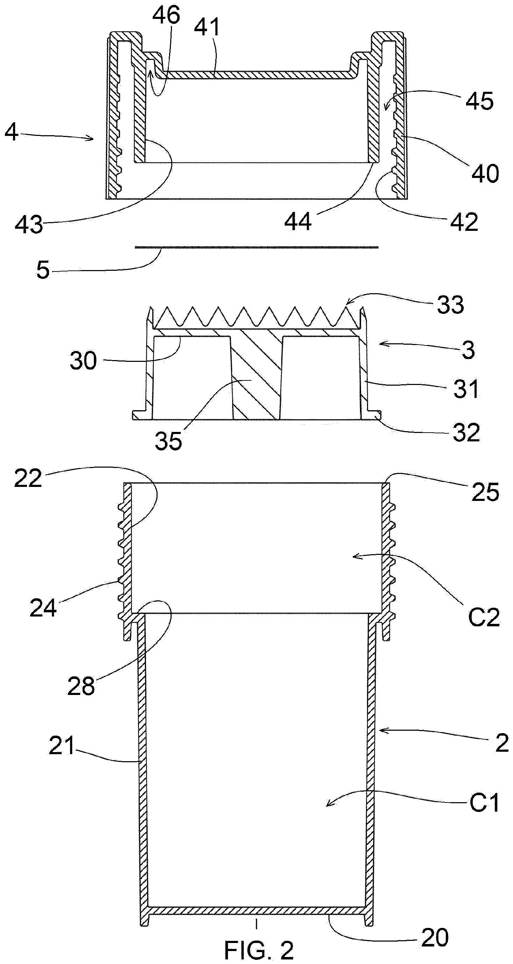

With reference to Figs. 1 and 2, the biopsy container assembly (1) comprises:
- a container (2),
- a crown (3) mounted inside the container (2), and
- a cap (4) applied on the container (2).

The container (2) has a substantially cylindrical shape, is internally empty and is open on top.

The container (2) has a bottom wall (20) and a lower lateral wall (21) that defines a lower chamber (C1). The lower lateral wall (21) is joined to an upper lateral wall (22) by means of a step-shaped collar (23) that projects outward from the lower lateral wall (21), acting as a shoulder. The upper lateral wall (22) has an external thread (24). The upper lateral wall (22) terminates with an upper edge (25). The upper lateral wall (22) forms an upper chamber (C2) that is open to the outside and communicates with the lower chamber (C1).

The diameter of the upper chamber (C2) is larger than the diameter of the lower chamber (C1). The height of the upper chamber (C2) is lower than the height of the lower chamber (C1). The volume of the lower chamber (C1) is larger than the volume of the upper chamber (C2).

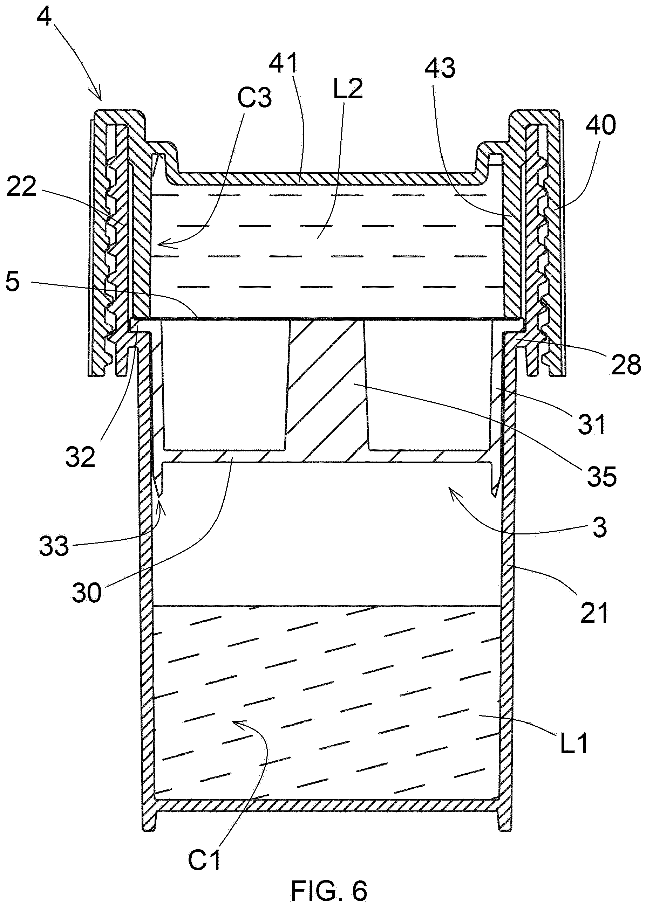

With reference to Fig. 6, the lower chamber (C1) is suitable for being partially filled with a buffer liquid (L1) consisting of a buffer solution, which is not harmful to the human body. For illustrative purposes, the level of the buffer liquid (L1) is lower than half the height of the lower lateral wall of the container.

The container (2) is made of a hard plastic material, such as injection molded polypropylene, preferably in one piece.

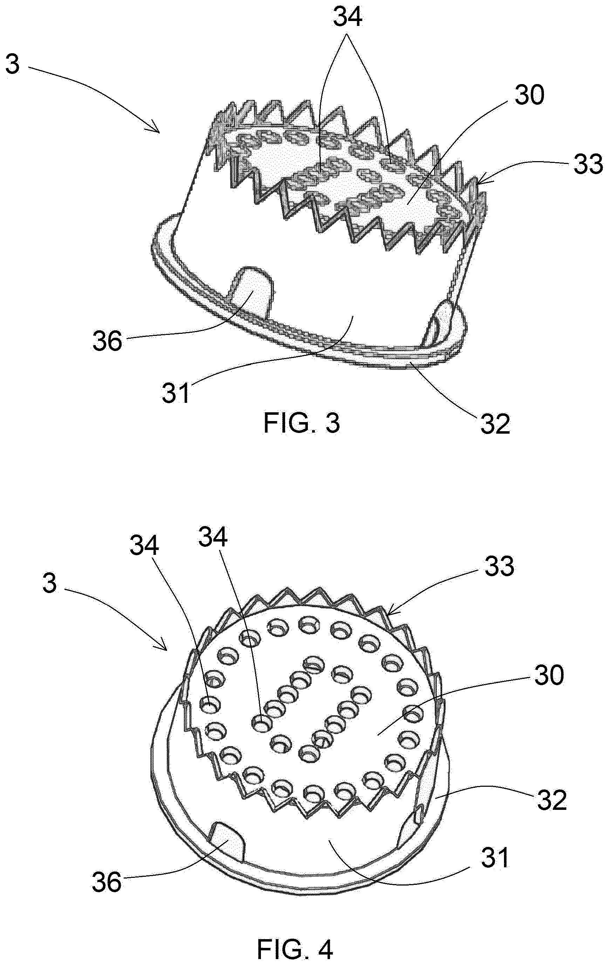

With reference to Figs. 3 and 4, the crown (3) comprises a base wall (30) with discoidal shape. A lateral wall (31) with cylindrical shape extends from the base wall (30). A collar (32) with annular shape protrudes radially outward from the lateral wall (31) of the crown. The collar (32) of the crown is suitable for abutting the collar (22) of the container. Therefore, the diameter of the collar (32) of the crown is lower than the internal diameter of the upper chamber (C2) and higher than the internal diameter of the lower chamber (C1).

The lateral wall (31) of the crown has an edge with a toothing (33) that protrudes with respect to the base wall (30). The toothing (33) may include triangular teeth. The toothing (33) is disposed in a first edge of the lateral wall of the crown and the collar (32) is disposed in a second edge of the lateral wall of the crown.

The base wall (30) of the crown comprises a plurality of holes (34) suitable for letting a liquid pass through. A cylindrical shank (35) protrudes axially from the base wall (30) of the crown, and is disposed coaxially inside the lateral wall (31) of the crown.

A plurality of apertures (36) is obtained in the lateral wall (31) of the crown near the collar (32).

The external diameter of the base wall (30) and of the lateral wall (31) of the crown is lower than the internal diameter of the lower chamber (C1) of the container. In view of the above, as shown in Fig. 6, the base wall (30) and the lateral wall of the crown are inserted in the lower chamber (C1) of the container until the collar (32) of the crown abuts the collar (28) of the container. In such a case, the toothing (33) of the crown is directed downward and the crown is in an initial position.

The height of the crown (3) is lower than half the height of the lower lateral wall (21) of the container. In this way, the crown is not in contact with the buffer fluid (L1) contained in the container.

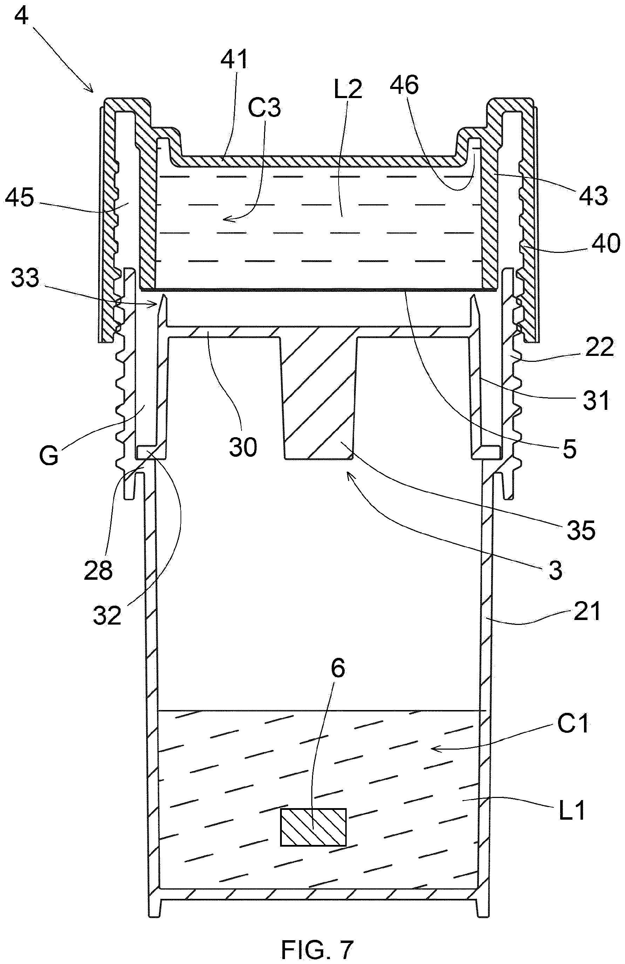

The crown (3) can be turned upside down (rotated by 180° relative to a horizontal axis) with respect to the initial position of Fig. 6. As shown in Fig. 7, when the crown is turned upside down, the crown is in an operating position wherein the toothing (33) of the crown is directed upward and the collar (32) of the crown abuts the collar (28) of the container. In such a situation, an annular empty space (G) with a width equal to the radial thickness of the collar (32) of the crown is generated between the lateral wall (31) of the crown and the upper lateral wall (22) of the container.

The crown (3) is made of a hard plastic material, such as injection molded polypropylene, preferably in one piece.

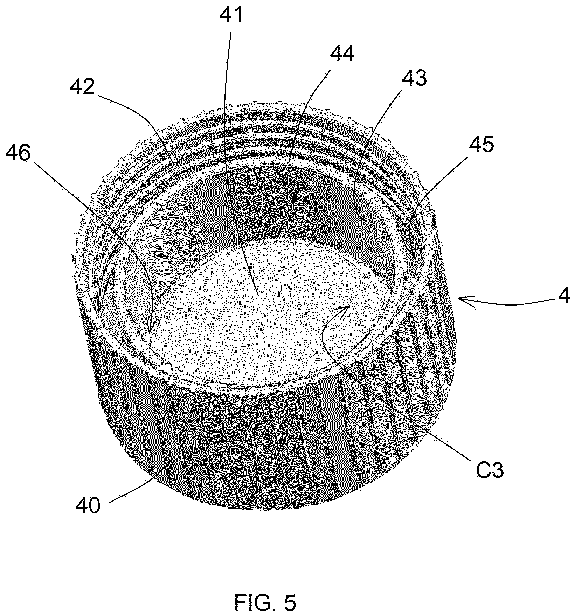

With reference to Figs. 2 and 5, the cap (4) has a substantially cylindrical shape and is open on the bottom. The cap has a lateral wall (40) joined to an upper wall (41).

The lateral wall (40) of the cap has an internal thread (42) suitable for being screwed on the external thread (24) of the container. An internal wall (43) with cylindrical shape protrudes in lower position from the upper wall (41) of the cap, coaxially inside the lateral wall (40) of the cap. The internal wall (43) has a lower edge (44).

The internal wall (43) and the upper wall (41) of the cap form a chamber (C3) suitable for containing a biopsy fluid (L2), such as 8% P/V formalin. As it is known, the exhalations of biopsy fluid are harmful to the human organism.

The cap (4) is made of a hard plastic material, such as injection molded polypropylene, preferably in one piece.

A membrane (5) is attached to the lower edge (44) of the internal wall of the cap so as to seal the biopsy fluid (L2) inside the chamber (C3) of the cap. For illustrative purposes, the membrane (5) may consist of an aluminum sheet that is co-extruded with polyolefins and may have a thickness comprised between 0.5 and 0.2 mm, preferably 0.1 mm. The membrane (5) may be heatsealed to the lower edge (44) of the internal wall of the cap.

The volume of the chamber (C3) of the cap is lower than half the volume of the chamber (C1) of the container, so that a mixture of the buffer fluid (L1) and of the biopsy fluid does not completely fill the chamber (C1) of the container.

An annular empty space (45) suitable for accommodating the upper lateral wall (22) of the container is provided between the internal wall (43) and the lateral wall (40) of the cap.

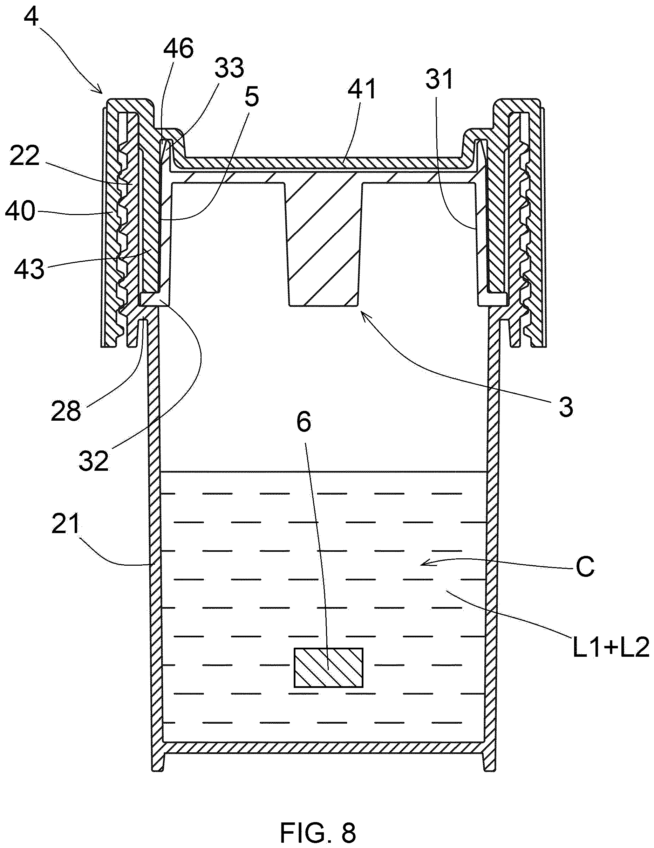

The thickness of the internal wall (43) of the cap is lower than the width of the empty space (G) between the lateral wall (31) of the crown and the upper lateral wall (22) of the container. In view of the above, the cylindrical wall (43) of the cap can fit into the empty space (G) between the lateral wall (31) of the crown and the upper lateral wall (22) of the container, as shown in Fig. 8.

The upper wall (41) of the cap has an annular groove (46) disposed internally relative to the internal wall (43). The annular groove (46) is suitable for accommodating the toothing (33) of the crown, when the crown is in operating position and the cap is closed, as shown in Fig. 8.

After screwing the cap (4), the holes (34) in the base wall of the crown abut the upper wall of the cap (41). In this way, the upper wall of the cap (41) closes the holes of the base wall of the crown, preventing the mixture of buffer fluid and biopsy fluid from going into an empty space provided between the base wall of the crown and the upper wall of the cap.

Following is a description of the operation of the biopsy container assembly (1).

With reference to Fig. 6, initially, the chamber (C1) of the container is partially filled with the buffer fluid (L1); the crown (3) is disposed in the container in the initial position with the toothing (33) directed downward; the biopsy fluid (L2) is contained in the chamber (C3) of the cap; and the internal thread (42) of the cap is screwed into the external thread (24) of the container.

The cap (4) is unscrewed and is removed from the container (2) and the crown (3) is removed from the container (1).

With reference to Fig. 7, a bioptic sample (6) is disposed in the buffer fluid (L1); the crown (3) is turned upside down and inserted into the container with the toothing (33) directed upward, and the internal thread (42) of the cap is screwed on the internal thread (24) of the container, causing the translation of the cap toward the container.

With reference to Fig. 8, the toothing (33) of the crown tears off the membrane (5) of the cap and, because of gravity and of the pressure due to the upward thrust of the crown (3), the biopsy fluid (L2) leaves the chamber (C3) of the cap, passes through the crown (3) and reaches the lower chamber (C1) of the container where it is mixed with the buffer fluid (L1). Otherwise said, the crown (3) acts as a plunger to ensure that all of the biopsy fluid (L2) leaves the chamber (C3) of the cap.

The container assembly (1) according to the invention has been designed to solve all problems of the container assemblies of the prior art, because the container assembly (1) can be used as a standard container:
- unscrewing of the cap (4) and easy introduction of any type of biopsy (6) in the buffer fluid (L1);
- screwing of the cap (4) with simultaneous activation of the crown (3) to break the membrane (5) and let the biopsy fluid (L2) flow out;
- the biopsy (6) is housed in a space devoid of empty spaces or slots;
- unscrewing of the cap (4) and easy extraction of the biopsy (6).

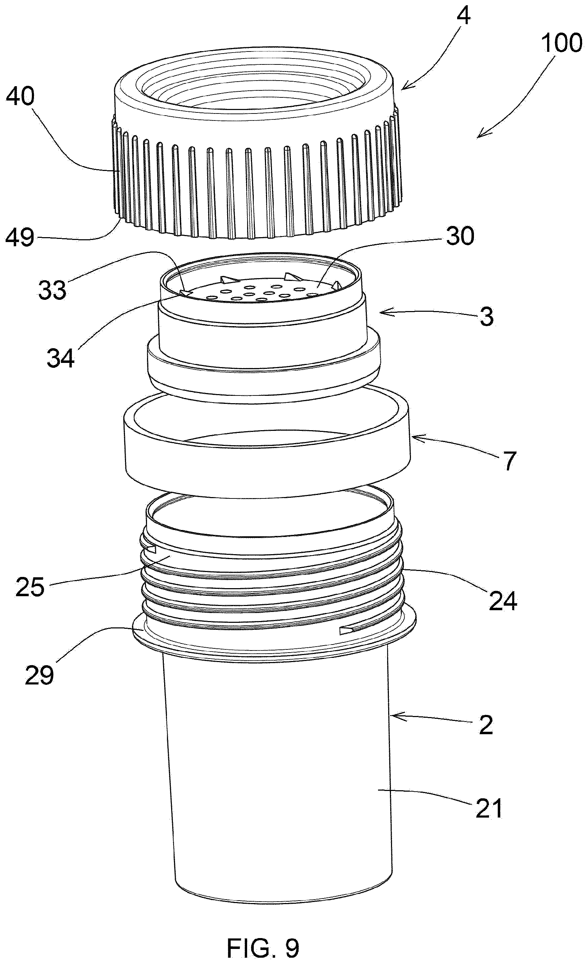

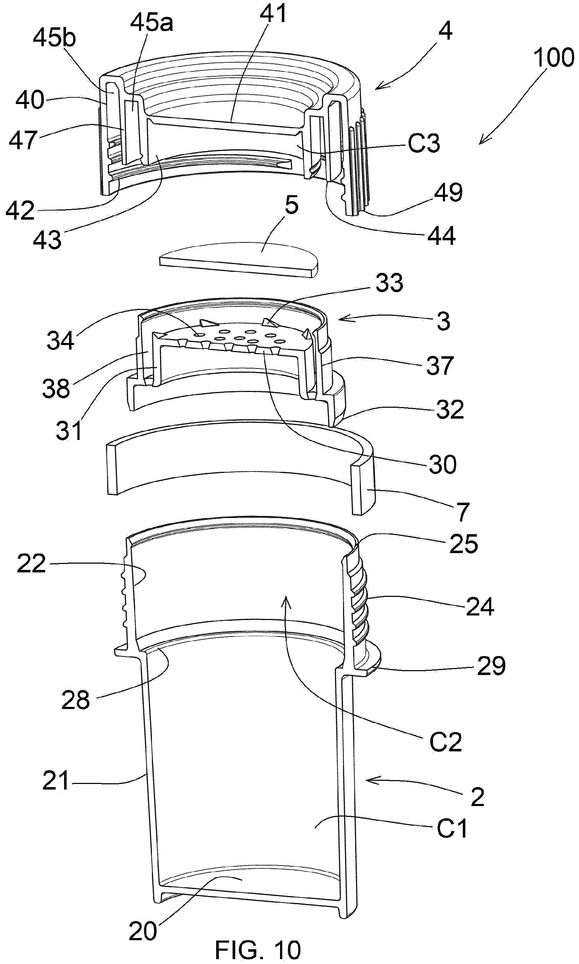

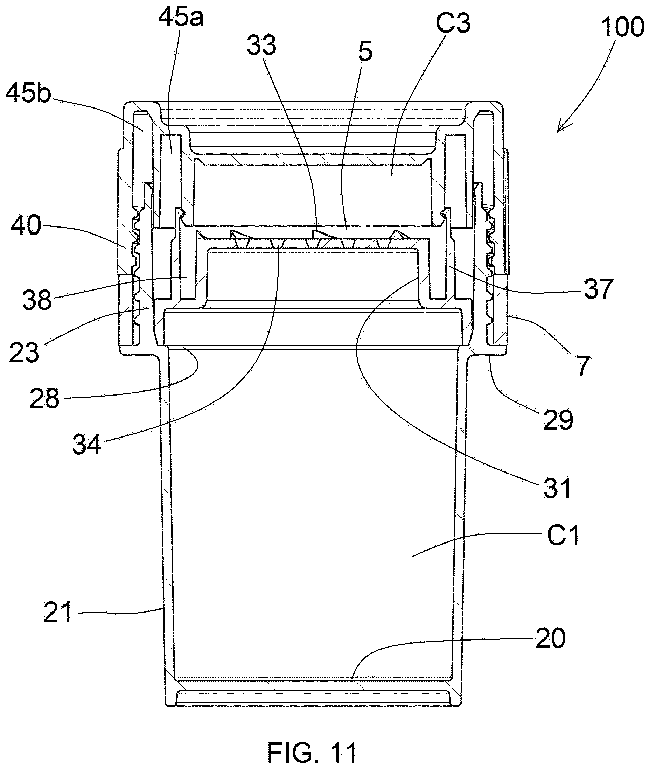

With reference to Figs. 9-11, a container assembly (100) according to a second embodiment is described.

In the following description, parts that are identical or correspond to the ones described above will be indicated with the same numerals, omitting their detailed description.

The biopsy container assembly (100) comprises
- a container (2),
- a crown (3) mounted in the container (2),
- a cap (4) applied on the container (2), and
- a safety strap (7) disposed between the container (2) and the cap (4) to prevent the lowering of the cap (4) on the container (2).

In particular, the container (2) has a collar (29) that protrudes externally under the thread (25) of the container. The lateral wall (40) of the cap (4) has a lower edge (49). The safety strap (7) is disposed on the collar (29) of the container. The lower edge (49) of the lateral wall (40) of the cap abuts the safety strap (7) that prevents the cap from being screwed and additionally lowered.

In such a case, the crown (3) is not rotated, and remains always with the toothing (33) directed upwards, namely towards the membrane (5). The safety strap (5) prevents the cap from being lowered and the toothing (33) of the crown from tearing off the membrane (5) before use. When the container assembly is to be used, the user removes the safety strap (7), screws the cap (4) onto the container (3) and the toothing (33) of the crown tears off the membrane (5) so that the biopsy fluid (L2) contained in the chamber (C3) of the cap falls in the chamber (C1) of the container with the buffer solution (L1).

In such a case, the crown (3) has an external lateral wall (37) that surrounds the lateral wall (31) of the crown so as to generate an annular empty space (38) that is open on top.

The cap (4) has an intermediate lateral wall (47) with cylindrical shape, disposed between the internal lateral wall (43) of the cap and the lateral wall (40) of the cap. In this manner, the cap (4) has a first annular empty space (45a) between the internal lateral wall (43) and the intermediate lateral wall (47) and a second annular empty space (45b) between the intermediate lateral wall (47) and the lateral wall (40).

When the cap (4) is screwed on the container (2), the internal lateral wall (43) of the cap enters the annular empty space (38) of the crown; the external lateral wall (37) of the crown enters the first annular empty space (45a) of the cap; and the upper lateral wall (22) of the container enters the second annular empty space (45b) of the cap.

It must be considered that in both embodiments of the container assemblies, because of the rupture of the membrane (5), the cap (4) is lowered and the upper wall (41) of the cap acts as a plunger to push the biopsy fluid (L2) through the holes (34) of the base wall of the crown. Thus, the biopsy fluid (L2) falls not only by gravity, but also because of the thrust of the cap.

In order to overcome the drawbacks of the prior art, after tearing off the membrane (5), the biopsy fluid (L2) in the chamber (C3) of the cap is pushed into the chamber (C1) of the container during the closing of the cap (4). It is essential that the proportion between biopsy liquid (L2) (concentrated formalin) and buffer solution (L1) is respected in order to provide a ready-to-use liquid.

At the same time, if the container is turned upside down, the potential passage of the bioptic sample (6) by means of reflux from the container (2) to the membrane (5), which is fragmented after being torn off, must be prevented.

In order to solve such a problem, in the container assembly according to the invention, the cap (4) acts as a plunger, wherein the upper wall (41) of the cap pushes the biopsy fluid (L2) through the holes (34) in the base wall (30) of the crown. In such a way, the holes (34) can be sufficiently small, for example with a diameter lower than 2 mm, preferably 1 mm, in such a way to prevent that the biopsy sample (6) passes through the base wall of the crown, reaches the ruptured membrane (5) and gets stuck in the membrane.

When screwing and lowering the cap (4), the membrane (5) abuts the toothing (33) of the crown, which cuts the membrane (5) on a peripheral edge of the membrane, pierces small holes in the membrane and breaks the membrane along its peripheral edge. In such a situation, the biopsy fluid (L2), which is sufficiently dense, will not fall into the holes (34) in the base wall (30) of the crown by gravity because said holes (34) are sufficiently small.

A subsequent screwing of the cap (4) will cause the penetration of the crown (3) in the chamber (C3) of the cap with the biopsy fluid (L2) and the upper wall (41) of the cap will cause a forced pressure on the biopsy fluid (L2) that forces the biopsy fluid (L2) to forcedly pass through the holes (34) disposed on the base wall of the crown.

The membrane (5), which has been previously detached by the toothing of the crown, will be accompanied by the crown towards an upper portion of the chamber (C3) of the cap, without interfering with the holes (34) of the base wall of the crown that allow for the passage of the biopsy liquid (L2) because the tolerance between the wall of the chamber (C3) of the cap and the circumference of the detached membrane (5) allows the passage of the biopsy liquid (L2). A complete screwing of the cap (4) will cause the crown (3) to abut the upper wall of the cap, and the base wall (30) of the crown will adhere to the upper wall (41) of the cap, closing all the holes (34) in the base wall of the crown under pressure. In such a way, it impossible even for very small samples to pass through the holes (34) in the base wall of the crown.

By simply screwing the cap (4), such a mechanism forces the entire amount of biopsy liquid (L2) to flow down under pressure, while simultaneously closing all the openings that may be a source of reflux for the tissue samples or for the cells, guaranteeing a correct concentration of the solution and maintaining all samples inside the container.

## Claims

1. Biopsy container assembly (1) comprising:
- a container (2) with a lower chamber (C1) suitable for containing a buffer liquid (L1),
- a cap (4) coupled with said container (2); said cap being provided with a chamber (C3) that contains a biopsy liquid (L2) sealed by means of a membrane (5), and
- a crown (3) with a toothing (33) suitable for tearing off said membrane (5) of the cap;
**characterized in that**
said crown (3) is suitable for being disposed in the container (2) in an initial position, wherein the toothing (33) is directed downwards in order not to interfere with the membrane (5) of the cap when the cap is closed on the container, and in an operating position, wherein the toothing (33) is directed upwards in order to tear off the membrane (5) of the cap when the cap is closed on the container.

2. The container assembly (1) of claim 1, wherein said container (1) comprises a bottom wall (20), a lower lateral wall (21) that defines said lower chamber (C1), and a collar (23) shaped like a step that projects outwards from the lower wall (21), acting as a shoulder; said collar (23) separates the lower chamber (C1) from an upper chamber (C2) and said crown (3) comprises a lateral wall (31) with said toothing (33) and a collar (32) that protrudes radially outwards from the lateral wall and abuts said collar (23) of the container.

3. The container assembly (1) of claim 2, wherein said toothing (33) is provided in a first edge of the lateral wall (31) of the crown and said collar (32) is provided in a second edge of the lateral wall of the crown.

4. The container assembly (1) of claim 3, wherein the external diameter of said lateral wall (31) of the crown is lower than the internal diameter of the lower lateral wall (21) of the container, and the external diameter of said collar (32) of the crown is higher than the internal diameter of the lower lateral wall (21) of the contained and lower than the internal diameter of the upper lateral wall (22) of the container.

5. The container assembly (1) according to any one of the preceding claims, wherein said cap (4) comprises a lateral wall (40) that protrudes in lower position from an upper wall (41) and an internal lateral wall (43) that is disposed coaxially inside the lateral wall (40) in such a way to define said chamber (C3) of the cap; said membrane (5) being sealed to a lower edge (44) of the internal lateral wall (43) of the cap.

6. The container assembly (1) of claim 4 and 5 wherein, when said crown (3) is in operating position, said collar (32) of the crown abuts the collar (28) of the container and an empty space (G) is formed between the lateral wall (31) of the crown and the upper lateral wall (22) of the container, said empty space (G) being suitable for receiving the internal lateral wall (43) of the cap.

7. The container assembly (1) according to any one of the preceding claims, wherein said cap (4) comprises an internal thread (42) that is screwed on an external thread (24) provided in said container.

8. The container assembly (1) according to any one of the preceding claims, wherein said crown (3) comprises a base (30) joined to said lateral wall (31); said base (30) having a plurality of holes (34) for the passage of said biopsy liquid (L2).

9. The container assembly (1) according to any one of the preceding claims, wherein said crown (3) comprises a shank (35) that projects from the base (30) coaxially to said lateral wall (31) of the crown.

10. The container assembly (1) according to any one of the preceding claims, wherein said membrane (5) is made of an aluminum sheet coextruded with polyolefins having a thickness of 0.5 - 0.2 mm and said toothing (33) of the crown comprises triangular teeth disposed along an edge of the lateral wall (31) of the crown.

## Patentansprüche

1. Biopsiebehälteranordnung (1), umfassend:
- einen Behälter (2) mit einer unteren Kammer (C1), die dazu geeignet ist, eine Pufferlösung (L1) aufzunehmen,
- einen Deckel (4), der mit dem Behälter (2) gekoppelt ist; wobei der Deckel mit einer Kammer (C3) versehen ist, die eine Biopsie-Flüssigkeit (L2) aufnimmt und mittels einer Membran (5) versiegelt ist, und
- eine Zahnkrone (3) mit einer Zähnung (33), die geeignet ist, die Membran (5) des Deckels zu durchlöchern;
**dadurch gekennzeichnet, dass**
die Zahnkrone (3) dazu geeignet ist, in dem Behälter (2) in einer Anfangsposition angeordnet zu werden, in der die Zähnung (33) nach unten gerichtet ist, um nicht mit der Membran (5) des Deckels zu interferieren, wenn der Deckel auf dem Behälter geschlossen wird, und in einer Betriebsposition, in der die Zähnung (33) nach oben gerichtet ist, um die Membran (5) des Deckels zu durchlöchern, wenn der Deckel auf dem Behälter geschlossen wird.

2. Behälteranordnung (1) nach Anspruch 1, wobei der Behälter (1) eine Bodenwand (20), eine untere Seitenwand (21), die die untere Kammer (C1) definiert, und einen Bund (23) in Form einer Stufe umfasst, die aus der unteren Wand (21) nach außen vorsteht und als Schulter fungiert; wobei der Bund (23) die untere Kammer (C1) von einer oberen Kammer (C2) trennt und die Zahnkrone (3) eine Seitenwand (31) mit der Zähnung (33) und einen Bund (32) umfasst, der aus der Seitenwand radial nach außen vorsteht und gegen den Bund (23) des Behälters anliegt.

3. Behälteranordnung (1) nach Anspruch 2, wobei die Zähnung (33) in einem ersten Rand der Seitenwand (31) der Zahnkrone herausgearbeitet ist und der Bund (32) in einem zweiten Rand der Seitenwand der Zahnkrone herausgearbeitet ist.

4. Behälteranordnung (1) nach Anspruch 3, wobei der Außendurchmesser der Seitenwand (31) der Zahnkrone kleiner als der Innendurchmesser der unteren Seitenwand (21) des Behälters ist und der Außendurchmesser des Bundes (32) der Zahnkrone größer als der Innendurchmesser der unteren Seitenwand (21) des Behälters ist und kleiner als der Innendurchmesser der oberen Seitenwand (22) des Behälters ist.

5. Behälteranordnung (1) nach einem der vorstehenden Ansprüche, wobei der Deckel (4) eine Seitenwand (40) umfasst, die unterseitig aus einer oberen Wand (41) vorsteht, und eine innere Seitenwand (43), die koaxial so in der Seitenwand (40) angeordnet ist, dass sie die Kammer (C3) des Deckels definiert; wobei die Membran (5) an einem unteren Rand (44) der inneren Seitenwand (43) des Deckels angeschweißt ist.

6. Behälteranordnung (1) nach Anspruch 4 und 5, wobei wenn die Zahnkrone (3) in Betriebsposition ist, der Bund (32) der Zahnkrone an dem Bund (28) des Behälters in Anschlag geht und zwischen der Seitenwand (31) der Zahnkrone und der oberen Seitenwand (22) des Behälters ein Zwischenraum (G) gebildet wird, wobei der Zwischenraum (G) dazu geeignet ist, die innere Seitenwand (43) des Deckels aufzunehmen.

7. Behälteranordnung (1) nach einem der vorstehenden Ansprüche, wobei der Deckel (4) ein Innengewinde (42) umfasst, das sich auf ein Außengewinde (24) verschraubt, das in dem Behälter vorgesehen ist.

8. Behälteranordnung (1) nach einem der vorstehenden Ansprüche, wobei der Zahnkranz (3) eine Basis (30) umfasst, die mit der Seitenwand (31) verbunden ist; wobei die Basis (30) eine Vielzahl von Löchern (34) für den Durchfluss der Biopsie-Flüssigkeit (L2) aufweist.

9. Behälteranordnung (1) nach einem der vorstehenden Ansprüche, wobei der Zahnkranz (3) einen Schaft (35) umfasst, der aus der Basis (30) koaxial zu der Seitenwand (31) des Zahnkranzes vorsteht.

10. Behälteranordnung (1) nach einem der vorstehenden Ansprüche, wobei die Membran (5) aus einer mit Polyolefinen coextrudierten Aluminiumfolie mit einer Dicke von 0,5 bis 0,2 mm hergestellt ist und die Zähnung (33) des Zahnkranzes dreieckige Zähne umfasst, die entlang eines Randes der Seitenwand (31) des Zahnkranzes angeordnet sind.

## Revendications

1. Groupe récipient (1) pour biopsie comprenant :
- un récipient (2) muni d'une chambre inférieure (C1) destinée à contenir un liquide tampon (L1),
- un bouchon (4) qui s'accouple avec ledit récipient (2) ; ledit bouchon ayant une chambre (C3), contenant un liquide pour biopsie (L2), scellée au moyen d'une membrane (5), et
- une couronne (3) ayant une denture (33) apte à déchirer ladite membrane (5) du bouchon ;
**caractérisé en ce que**
ladite couronne (3) est apte à être disposée dans le récipient (2) dans une position initiale avec la denture (33) orientée vers le bas pour ne pas interférer avec la membrane (5) du bouchon lorsque le bouchon est fermé sur le récipient et dans une position opérationnelle avec la denture (33) orientée vers le haut pour déchirer la membrane (5) du bouchon lorsqu'on ferme le bouchon sur le récipient.

2. Groupe récipient (1) selon la revendication 1, où ledit récipient (1) comprend une paroi de fond (20), une paroi latérale inférieure (21) qui définit ladite chambre inférieure (C1) et un collier (23) en forme de gradin qui déborde vers l'extérieur de la paroi inférieure (21), en fonctionnant en tant qu'épaulement ; ledit collier (23) sépare la chambre inférieure (C1) d'une chambre supérieure (C2) et ladite couronne (3) comprend une paroi latérale (31) ayant ladite denture (33) et un collier (32) qui déborde radialement vers l'extérieur de la paroi latérale pour aller en butée sur ledit collier (23) du récipient.

3. Groupe récipient (1) selon la revendication 2, où ladite denture (33) est réalisée dans un premier bord de la paroi latérale (31) de la couronne et ledit collier (32) est réalisé dans un second bord de la paroi latérale de la couronne.

4. Groupe récipient (1) selon la revendication 3, où ladite paroi latérale (31) de la couronne a un diamètre externe inférieur par rapport au diamètre interne de la paroi latérale inférieure (21) du récipient et ledit collier (32) de la couronne a un diamètre externe supérieur du diamètre interne de la paroi latérale inférieure (21) du récipient et inférieur du diamètre interne de la paroi latérale supérieure (22) du récipient.

5. Groupe récipient (1) selon l'une quelconque des revendications précédentes, où ledit bouchon (4) comprend une paroi latérale (40) qui déborde inférieurement d'une paroi supérieure (41) et une paroi latérale interne (43) disposée de manière coaxiale dans la paroi latérale (40) en définissant ainsi ladite chambre (C3) du bouchon ; ladite membrane (5) étant scellée à un bord inférieur (44) de la paroi latérale interne (43) du bouchon.

6. Groupe récipient (1) selon les revendications 4 et 5, où quand ladite couronne (3) est en position opérationnelle, ledit collier (32) de la couronne est en butée sur le collier (28) du récipient et entre la paroi latérale (31) de la couronne et la paroi latérale supérieure (22) du récipient, il y a un écartement (G) apte à accueillir la paroi latérale interne (43) du bouchon.

7. Groupe récipient (1) selon l'une quelconque des revendications précédentes, où ledit bouchon (4) comprend un filetage interne (42) qui se visse sur un filetage externe (24) prévu dans ledit récipient.

8. Groupe récipient (1) selon l'une quelconque des revendications précédentes, où ladite couronne (3) comprend une base (30) reliée à ladite paroi latérale (31) ; ladite base (30) ayant une pluralité d'orifices (34) pour le passage du susdit liquide pour biopsie (L2).

9. Groupe récipient (1) selon l'une quelconque des revendications précédentes, où ladite couronne (3) comprend une tige (35) qui déborde de la base (30) de manière coaxiale par rapport à ladite paroi latérale (31) de la couronne.

10. Groupe récipient (1) selon l'une quelconque des revendications précédentes, où ladite membrane (5) est réalisée moyennant une feuille d'aluminium coextrudée avec polyoléfines ayant une épaisseur de 0,5 - 0,2 mm et ladite denture (33) de la couronne comprend des dents triangulaires disposées le long d'un bord de la paroi latérale (31) de la couronne.
